(19) Europäisches Patentamt
European Patent Office
Office européen des brevets

(11) Veröffentlichungsnummer: **0 668 282 A1**

# (12) EUROPÄISCHE PATENTANMELDUNG

(21) Anmeldenummer: **95101686.4**

(22) Anmeldetag: **08.02.95**

(51) Int. Cl.6: **C07D 498/10**, C07D 519/00, C08G 73/06, //(C07D498/10, 263:00,221:00),(C07D519/00, 498:00,498:00)

(30) Priorität: **19.02.94 DE 4405388**

(43) Veröffentlichungstag der Anmeldung: **23.08.95 Patentblatt 95/34**

(84) Benannte Vertragsstaaten: **AT BE CH DE ES FR GB IT LI NL PT SE**

(71) Anmelder: **HOECHST AKTIENGESELLSCHAFT**
**Brüningstrasse 50**
**D-65929 Frankfurt am Main (DE)**

(72) Erfinder: **Gaa, Karl, Dr.**
**Weinbergstrasse 2**
**D-89349 Burtenbach (DE)**
Erfinder: **Zäh, Matthias, Dr.**
**Siedlerstrasse 15**
**D-86368 Gersthofen (DE)**

## (54) Verfahren zur Herstellung von Polyalkyl-1-oxadiazaspirodecan-Verbindungen.

(57) Die Erfindung bezieht sich auf ein Verfahren zur Herstellung von Polyalkyl-1-oxa-diazaspirodecan-Verbindungen, die als hochwirksame Lichtschutzmittel für Polymere verwendet werden können. Die Reaktion erfolgt in einem inerten organischen Lösemittel in Anwesenheit von festem Alkalimetallhydroxid oder der entsprechenden Menge einer Mischung aus festem Alkalimetallhydroxid und Wasser als alleinigem Katalysator. Das Verfahren bietet den Vorteil, daß durch Änderung der Reaktionsführung in einem Mehrphasensystem und Verzicht auf einen Phasentransferkatalysator gleich gute Ergebnisse hinsichtlich Produktqualität und Ausbeute erreicht werden. Außerdem kann die Reaktionszeit von größer 6 Stunden auf 30 Minuten verringert werden. Dies führt zu einer erheblichen Steigerung der Raum-Zeit-Ausbeute und trägt somit zusätzlich zu einer Steigerung der Wirtschaftlichkeit des erfindungsgemäßen Verfahrens bei.

EP 0 668 282 A1

Die Erfindung bezieht sich auf ein Verfahren zur Herstellung von Polyalkyl-1-oxa-diazaspirodecan-Verbindungen, die als hochwirksame Lichtschutzmittel für Polymere verwendet werden können.

Verbindungen der Formel

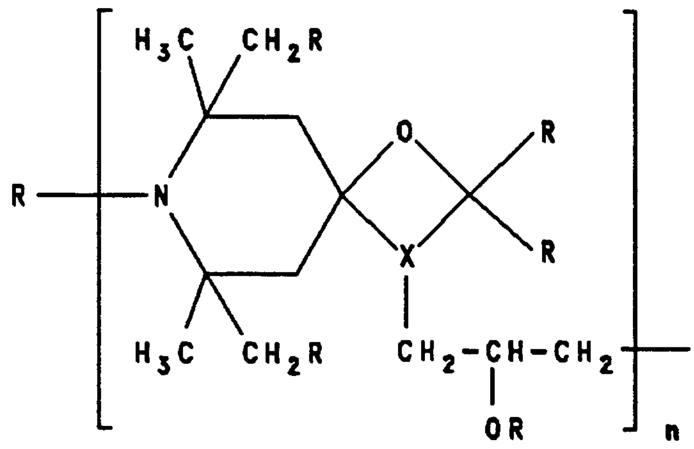

sind bekannt (vgl. EP 402 889). Das Herstellverfahren für diese Verbindungen ist dadurch gekennzeichnet, daß die Synthese in einem inerten Lösemittel in Anwesenheit von festem oder wässerigem Alkalimetallhydroxid und zusätzlich einem Phasentransferkatalysator erfolgt. Der Zusatz eines Phasentransferkatalysators hat jedoch den Nachteil, daß er bei der Aufarbeitung des Reaktionsgemisches in das Abwasser gelangt und somit eine Umweltbelastung darstellt. Insbesondere die als besonders wirksam beschriebenen quartären Ammonium- oder Phosphoniumhalogenide machen die Einleitung des Abwassers in eine biologische Kläranlage unmöglich, da quartäre Ammonium- und Phosphoniumsalze bakterizide Wirkung besitzen und nicht in einer biologischen Kläranlage aufgearbeitet werden können. Das Abwasser muß daher aufwendig als Sondermüll entsorgt werden.

Es bestand somit die Aufgabe, ein Verfahren zu finden, das die genannten Verbindungen bei möglichst kurzen Reaktionszeiten in möglichst hohen Ausbeuten bei mindestens gleicher Produktqualität möglich macht, ohne dabei die aus dem Stand der Technik bekannten Nachteile einer zu geringen Umweltverträglichkeit und einer dadurch bedingten aufwendigen Abwasserentsorgung aufzuweisen.

Es wurde gefunden, daß die Aufgabe gelöst werden kann, wenn man zur Herstellung der genannten Verbindungen als alleinigen Katalysator festes oder wässeriges Alkalimetallhydroxid verwendet und die Reaktion bei 90 bis 95 °C durchführt.

Die vorliegende Erfindung betrifft somit ein Verfahren zur Herstellung von Polyalkyl-1-oxa-diazaspirodecan-Verbindungen der Formel I

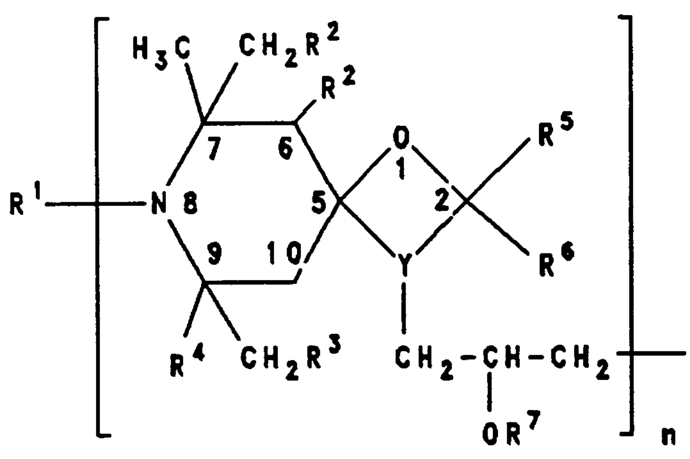

(I),

worin

n eine ganze Zahl von 1 bis 50 ist und

Y die Bedeutung einer Gruppe der Formel II oder III

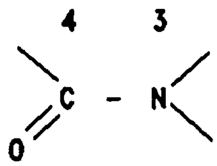 (II) 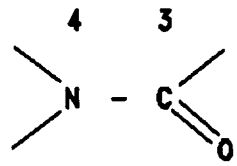 (III)

hat, wobei die Indices 3 bzw. 4 die Ringpositionen im Diazaspirodecansystem angeben und eine Bindung des Stickstoffs mit einer $CH_2$-Gruppe der Propylen-2-oxygruppe verknüpft ist,

$R^1$ ein Wasserstoffatom, ein Sauerstoffatom, eine NO-Gruppe, eine $C_1$-$C_{12}$- - Alkylgruppe, eine Allylgruppe, eine $C_1$-$C_{22}$-Acylgruppe, eine Benzylgruppe, eine $C_1$-$C_{12}$-Alkyloxy-Gruppe oder eine $C_3$-$C_{12}$-Cycloalkoxy-Gruppe,

$R^2$ und $R^3$ entweder gleich sind und ein Wasserstoffatom oder eine $C_1$-$C_5$-Alkylgruppe bedeuten, wobei dann $R^4$ eine Methylgruppe ist, oder

$R^2$ ein Wasserstoffatom oder eine $C_1$-$C_5$-Alkylgruppe ist und $R^3$ und $R^4$ zusammen mit den sie verbindenden Kohlenstoffatomen eine $C_5$- oder $C_6$-Cycloalkylgruppe oder eine Gruppe der Formel

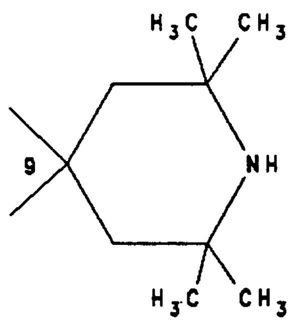

bilden,

$R^5$ und $R^6$ gleich oder verschieden sind und für ein Wasserstoffatom, eine $C_1$-$C_{30}$-, - Alkylgruppe, für eine unsubstituierte oder durch Chlor oder $C_1$-$C_4$-Alkyl substituierte $C_7$-$C_{12}$-Phenylalkylgruppe stehen, oder

$R^5$ und $R^6$ zusammen mit dem sie verbindenden Kohlenstoffatom eine unsubstituierte oder durch bis zu vier $C_1$-$C_4$-Alkylgruppen substituierte $C_5$-$C_{18}$-Cycloalkylgruppe oder eine Gruppe der Formel

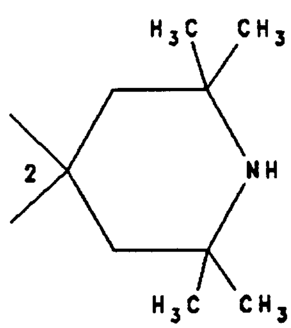

bilden,

$R^7$ bei n = 1 keine Bedeutung hat, sodaß das Sauerstoffatom mit der endständigen $CH_2$-Gruppe verbunden ist und einen Oxiranring bildet,

$R^7$ bei n > 1 ein Wasserstoffatom oder eine $C_1$-$C_{22}$-Acylgruppe ist, oder in der endständigen Monomereneinheit keine Bedeutung hat, so daß das Sauerstoffatom mit der enständigen $CH_2$-Gruppe verbunden ist

und einen Oxiranring bildet, durch Umsetzung einer Verbindung der Formel IV

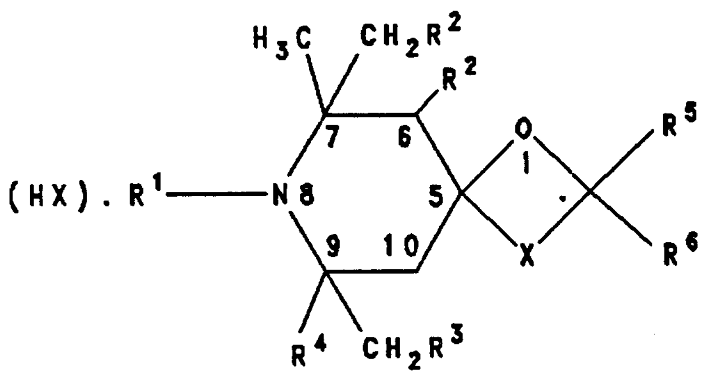

(IV)

worin $R^1$, $R^2$, $R^3$, $R^4$, $R^5$ und $R^6$ die obengenannte Bedeutung haben und (HX) ein Säurerest ist, oder deren Salz mit einer Protonensäure
mit einem Epihalohydrin der Formel V

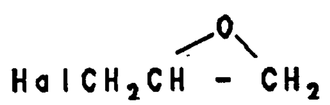

(V)

worin Hal ein Chlor-, Brom- oder Jodatom ist, im Molverhältnis 1:1 bis 1:10 in Anwesenheit eines Alkalihydroxyds in einem inerten organischen Lösemittel und, bei n > 1, Erhitzen der entstandenen Epoxiverbindung VI

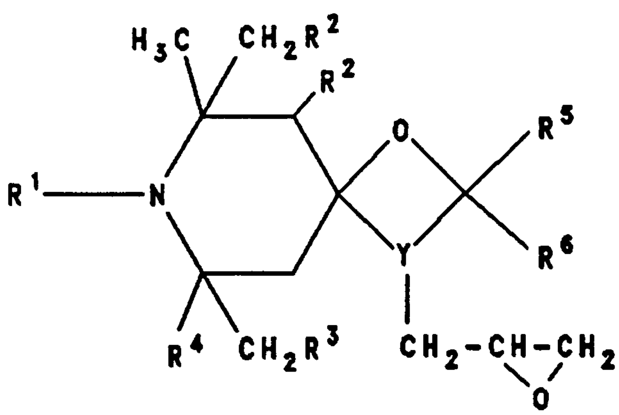

(VI)

worin $R^1$, $R^2$, $R^3$, $R^4$, $R^5$ und $R^6$ die obengenannte Bedeutung haben,
auf eine Temperatur von 100 bis 240 °C, dadurch gekennzeichnet, daß die Umsetzung der Verbindung der Formel IV mit der Verbindung der Formel V in Gegenwart der äquimolaren bis zwanzigfach molaren Menge festen Alkalimetallhydroxids oder der entsprechenden Menge einer Mischung aus festem Alkalimetallhydroxid und Wasser im Gewichtsverhältnis 1:9 bis 9:1 als alleinigem Katalysator durchgeführt wird und nach Beendigung der Reaktion dem Gemisch soviel Wasser zugesetzt wird, daß sich zwei Phasen bilden.

In der Formel (I) der erfindungsgemäß herzustellenden Polyalkyl-1-oxa-diazaspirodecan-Verbindungen

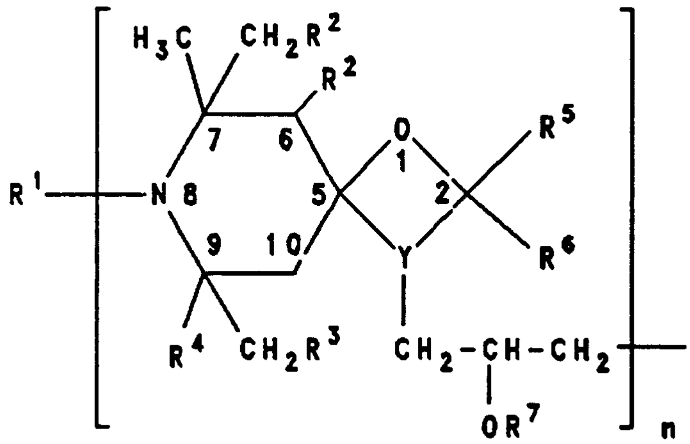

(I),

bedeutet

n eine ganze Zahl von 1 bis 50 , vorzugsweise 1 bis 15, insbesondere 1 bis 7.

Y hat die Bedeutung einer Gruppe der Formel II oder III

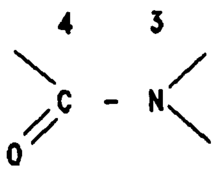

(II)

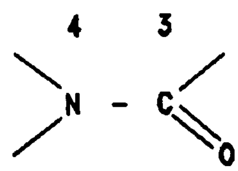

(III)

wobei die Indices 3 bzw. 4 die Ringpositionen im Diazaspirodecansystem angeben und eine Bindung des Stickstoffs mit einer $CH_2$-Gruppe der Propylen-2-oxygruppe verknüpft ist.

$R^1$ bedeutet ein Wasserstoffatom, ein Sauerstoffatom, eine NO-Gruppe, eine $C_1$-$C_{12}$-, vorzugsweise $C_1$-$C_4$-Alkylgruppe, eine Allylgruppe, eine $C_1$-$C_{22}$-Acylgruppe, vorzugsweise Acetylgruppe, eine Benzylgruppe, eine $C_1$-$C_{12}$-, vorzugsweise $C_1$-$C_4$-Alkyloxy-Gruppe oder eine $C_3$-$C_{12}$-, vorzugsweise $C_3$-$C_6$-Cycloalkoxy-Gruppe.

$R^2$ und $R^3$ sind entweder gleich und bedeuten ein Wasserstoffatom oder eine $C_1$-$C_5$-Alkylgruppe, vorzugsweise ein Wasserstoffatom, wobei dann $R^4$ eine Methylgruppe ist, oder

$R^2$ ist ein Wasserstoffatom oder eine $C_1$-$C_5$-Alkylgruppe und $R^3$ und $R^4$ bilden zusammen mit den sie verbindenden Kohlenstoffatomen eine $C_5$- oder $C_6$-Cycloalkylgruppe oder eine Gruppe der Formel

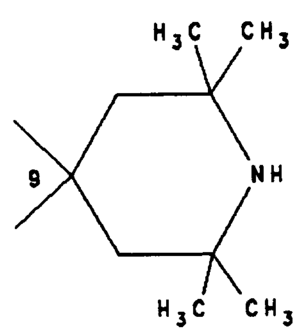

$R^5$ und $R^6$ sind gleich oder verschieden und stehen für ein Wasserstoffatom, eine $C_1$-$C_{30}$-, vorzugsweise $C_1$-$C_{18}$-Alkylgruppe, für eine unsubstituierte oder durch Chlor oder $C_1$-$C_4$-Alkyl substituierte $C_7$-$C_{12}$-Phenylalkylgruppe, oder

$R^5$ und $R^6$ bilden zusammen mit dem sie verbindenden Kohlenstoffatom eine unsubstituierte oder durch bis zu vier $C_1$-$C_4$-Alkylgruppen substituierte $C_5$-$C_{18}$-Cycloalkylgruppe oder eine Gruppe der Formel

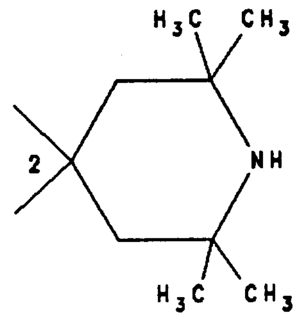

Bei n = 1 hat $R^7$ keine Bedeutung, sodaß das Sauerstoffatom mit der endständigen $CH_2$-Gruppe verbunden ist und einen Oxiranring bildet.
Bei n > 1 ist $R^7$ ein Wasserstoffatom oder eine $C_1$-$C_{22}$-, vorzugsweise $C_1$-$C_{12}$-Acylgruppe, oder hat in der endständigen Monomereneinheit keine Bedeutung, so daß das Sauerstoffatom mit der endständigen $CH_2$-Gruppe verbunden ist und einen Oxiranring bildet.

Die Herstellung der Verbindungen der Formel (I) erfolgt nach folgendem Reaktionsschema:

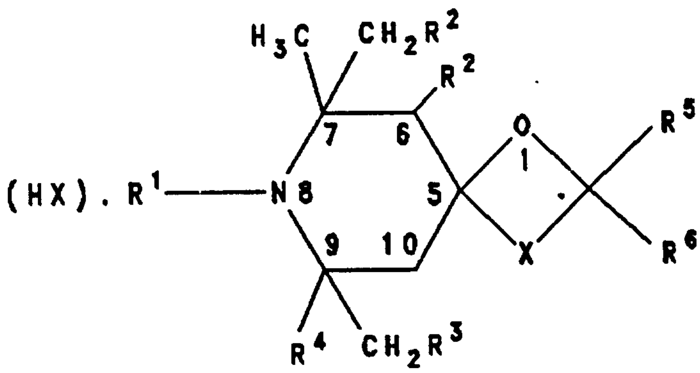

(IV)

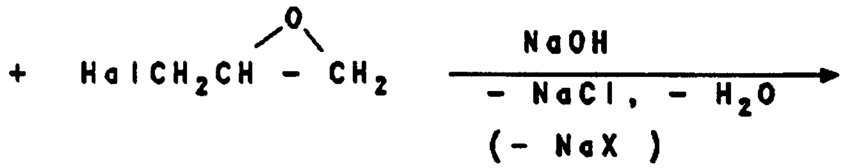


(V)

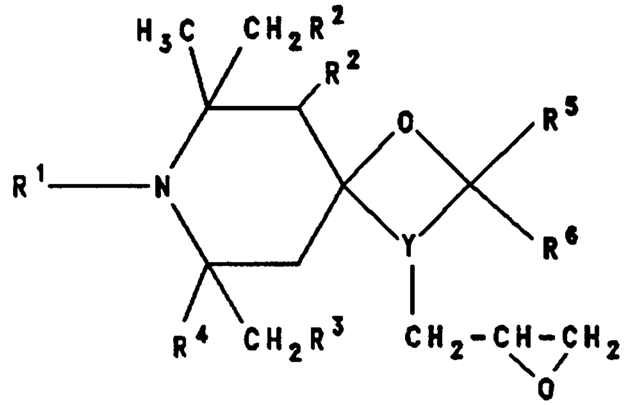

(VI)

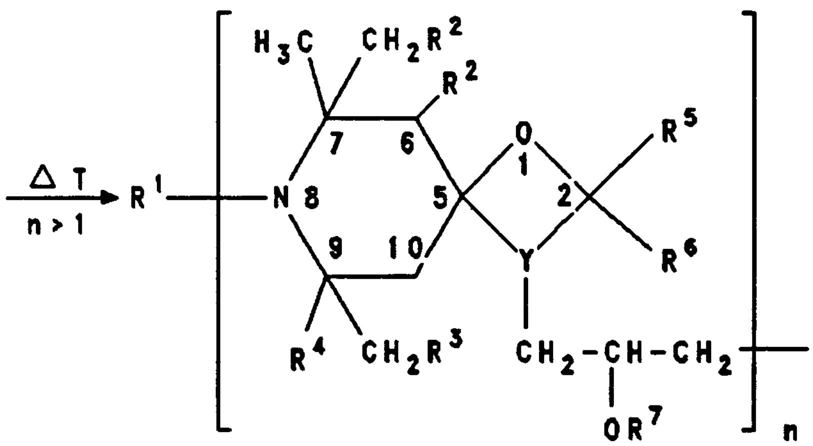


(I),

In den Formeln des Reaktionsschemas haben die Reste $R^2$, $R^3$, $R^4$, $R^5$, $R^6$, Y, Hal und n die oben angegebenen Bedeutungen: Der Rest $R^1$ bedeutet Wasserstoff und der Rest $R^7$ bedeutet ebenfalls Wasserstoff oder hat in der endständigen Monomereneinheit keine Bedeutung, so daß das Sauerstoffatom mit der endständigen $CH_2$-Gruppe einen Oxiranring bildet.

Geeignete Verbindungen der Formel IV sind beispielsweise

2-iso-Propyl-7,7,9,9,-tetramethyl-1-oxa-3,8-diaza-4-oxo-spiro-[4,5]-decan, 2-Butyl-7,7,9,9-tetramethyl-1-oxa-3,8-diaza-4-oxo-spiro-[4,5]-decan, 2-iso-Butyl-7,7,9,9-tetramethyl-1-oxa-3,8-diaza-4-oxo-spiro-[4,5]-decan, 2-Pentyl-7,7,9,9-tetramethyl-1-oxa-3,8-diaza-4-oxo-spiro-[4,5]-decan, 2-Hexyl-7,7,9,9-tetramethyl-1-oxa-3,8-diaza-4-oxo-spiro-[4,5]-decan, 2-Heptyl-7,7,9,9-tetramethyl-1-oxa-3,8-diaza-4-oxo-spiro-[4,5]-decan, 2-iso-Heptyl-7,7,9,9-tetramethyl-1-oxa-3,8-diaza-4-oxo-spiro-[4,5]-decan, 2-iso-Octyl-7,7,9,9-tetramethyl-1-oxa-3,8-diaza-4-oxo-spiro-[4,5]-decan, 2-Nonyl-7,7,9,9-tetramethyl-1-oxa-3,8-diaza-4-oxo-spiro-[4,5]-decan, 2-iso-Nonyl-7,7,9,9-tetramethyl-1-oxa-3,8-diaza-4-oxo-spiro-[4,5]-decan, 2-Undecyl-7,7,9,9-tetramethyl-1-oxa-3,8-diaza-4-oxo-spiro-[4,5]-decan, 2-Phenyl-7,7,9,9-tetramethyl-1-oxa-3,8-diaza-4-oxo-spiro-[4,5]-decan, 2-(4-Chlor-phenyl)-7,7,9,9-tetramethyl-1-oxa-3,8-diaza-4-oxo-spiro-[4,5]-decan, 2,2-Dimethyl-7,7,9,9-tetramethyl-1-oxa-3,8-diaza-4-oxo-spiro-[4,5]-decan, 2-Ethyl-2,7,7,9,9-pentamethyl-1-oxa-3,8-diaza-4-oxo-spiro-[4,5]-decan, 2-Propyl-2,7,7,9,9-pentamethyl-1-oxa-3,8-diaza-4-oxo-spiro-[4,5]-decan, 2-iso-Propyl-6,7,7,9,9-pentamethyl-1-oxa-3,8-diaza-4-oxo-spiro-[4,5]-decan, 2-Butyl-2,7,7,9,9-pentamethyl-1-oxa-3,8-diaza-4-oxo-spiro-[4,5]-decan, 2-iso-Butyl-2,7,7,9,9-pentamethyl-1-oxa-3,8-diaza-4-oxo-spiro-[4,5]-decan, 2-Pentyl-6,7,7,9,9-pentamethyl-1-oxa-3,8-diaza-4-oxo-spiro-[4,5]-decan, 2-Hexyl-2,7,7,9,9-pentamethyl-1-oxa-3,8-diaza-4-oxo-spiro-[4,5]-decan, 2-Nonyl-2,7,7,9,9-pentamethyl-1-oxa-3,8-diaza-4-oxo-spiro-[4,5]-decan, 2,2-Diethyl-7,7,9,9-tetramethyl-1-oxa-3,8-diaza-4-oxo-spiro-[4,5]-decan, 2,2-Dipropyl-7,7,9,9-tetramethyl-1-oxa-3,8-diaza-4-oxo-spiro-[4,5]-decan, 2,2-Dibutyl-7,7,9,9-tetramethyl-1-oxa-3,8-diaza-4-oxo-spiro-[4,5]-decan, 2-Ethyl-2-Pentyl-7,7,9,9-tetramethyl-1-oxa-3,8-diaza-4-oxo-spiro-[4,5]-decan, 2,2-Dibenzyl-7,7,9,9-tetramethyl-1-oxa-3,8-diaza-4-oxo-spiro-[4,5]-decan, 2,2,4,4-Tetramethyl-7-oxa-3,12-diaza-14-oxo-dispiro-[5,1,4,2]-tetradecan, 2,2,4,4-Tetramethyl-7-oxa-3,20-diaza-21-oxo-dispiro-[5,1,11,2]-heneicosan, 2,2,4,4-Tetramethyl-7-oxa-3,14-diaza-15-oxo-dispiro-[5,5,5,2]-pentadecan, 2,2,4,4,10,10,12,12-Octamethyl-7-oxa-3,11,14-triaza-15-oxo-dispiro-[5,1,5,2]-pentadecan, 2-Ethyl-2,7,7,9,9-pentamethyl-1-oxa-3,8-diaza-4-oxo-8-oxyl-spiro-[4,5]-decan.

Die als Ausgangsprodukte verwendeten Polyalkyloxadiazaspirodecane sind bekannt und nach den in US 4 110 334 und US 4 107 139 angegebenen Vorschriften zugänglich.

Besonders bevorzugt unter den Verbindungen IV ist 2,2,4,4-Tetramethyl-7-oxa-3,20-diaza-21-oxo-dispiro-[5,1,11,2]-heneicosan oder dessen Hydrochlorid.

Die Verbindung der Formel IV wird mit einem Epihalogenhydrin der Formel V, worin unter Hal ein Chlor-, Brom- oder Jodatom, bevorzugt Chlor, zu verstehen ist, im Molverhältnis 1:1 bis 1:10, vorzugsweise 1:1 bis 1:5 und insbesondere 1:1 bis 1:3 umgesetzt. Die Reaktion erfolgt in einem inerten organischen Lösemittel in Anwesenheit der äquimolaren bis zwanzigfach molaren Menge festen Alkalimetallhydroxids oder der entsprechenden Menge einer Mischung aus festem Alkalimetallhydroxid und Wasser im Gewichtsverhältnis 1:9 bis 9:1, vorzugsweise 4:6 bis 8:2, und insbesondere 5:5 bis 7:3.

Die Reaktionstemperatur liegt im Bereich von 20 bis 220 °C, vorzugsweise 60 bis 120 °C und insbesondere 80 bis 100 °C.

Als organisches Lösemittel kommt ein aliphatischer oder aromatischer Kohlenwasserstoff, wie beispielsweise Petrolether, Hexan, Heptan, Benzinschnitte, Toluol, Cyclohexan, Xylol etc. in Frage.

Die Reaktion ist im allgemeinen nach 30 bis 60 Minuten beendet.

Danach wird soviel Wasser zu der Reaktionsmischung gegeben, daß sich zwei Phasen bilden. Die Phasen werden getrennt, die organische Phase wird mehrfach mit Wasser gewaschen und das Lösemittel abdestilliert, wobei das Produkt zugleich azeotrop getrocknet wird.

Das dabei meist ölig anfallende Epoxid VI läßt sich isolieren (wenn $n = 1$) oder ohne weitere Reinigung durch Erhitzen auf 100 bis 240 °C, vorzugsweise 120 bis 220 °C und insbesondere 150 bis 200 °C in ein festes, amorphes, zunächst glasartig anfallendes Polymer I mit $n > 1$ überführen. Niedrige Polymerisationsgrade können durch kurze und hohe Polymerisationsgrade durch lange Polymerisierdauer erzielt werden. Weiterhin besteht bei gleicher Polymerisationsdauer mit steigender Temperatur die Tendenz zu höheren Polymerisationsgraden. Bei unter gleichen Polymerisationsbedingungen erhaltenen Produkten ist die Lösungsviskosität abhängig vom Grad der Umsetzung von Verbindung IV mit dem Epihalohydrin V und ist somit ein Maß für die Reinheit des Epoxids VI vor der Polymerisation.

Nach der Polymerisation kann das Polymer - wenn gewünscht - an den Positionen $R^1$ und $R^7$ des Moleküls nach an sich bekannten Methoden derivatisiert werden.

Die nach erfindungsgemäßem Verfahren hergestellten Verbindungen dienen als Lichtstabilisatoren in organischen Polymeren, beispielsweise in den nachstehend aufgeführten:

1. Polymere von Mono- und Diolefinen, beispielsweise Polyethylen hoher, mittlerer oder niederer Dichte (das gegebenenfalls vernetzt sein kann), Polypropylen, Polyisobutylen, Polybuten-1, Polymethylpenten-1, Polyisopren oder Polybutadien sowie Polymerisate von Cycloolefinen, wie beispielsweise von Cyclopenten oder Norbornen.

2. Mischungen der unter 1) genannten Polymeren, beispielsweise Mischungen von Polypropylen mit Polyethylen oder mit Polyisobutylen.

3. Copolymere von Mono- und Diolefinen untereinander oder mit anderen Vinylmonomeren, wie beispielsweise Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly(p-methylstyrol).

5. Copolymere von Styrol oder $\alpha$-Methylstyrol mit Dienen oder Acrylderivaten, wie beispielsweise Styrol-Butadien, Styrol-Maleinsäureanhydrid, Styrol-Acrylnitril, Styrol-Ethylmethacrylat, Styrol-Butadien-Ethylacrylat, Styrol-Acrylnitril-Methacrylat; Mischungen von hoher Schlagzähigkeit aus Styrol-Copolymeren und einem anderen Polymer, wie beispielsweise einem Polyacrylat, einem Dien-Polymeren oder einem Ethylen-Propylen-Dien-Terpolymeren; sowie Block-Copolymere des Styrols, wie beispielsweise Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie beispielsweise Styrol auf Polybutadien, Styrol und Acrylnitril auf Polybutadien, Styrol und Maleinsäureanhydrid auf Polybutadien, Styrol und Alkylacrylate oder Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, die beispielsweise als sogenannte ABS-, MBS-, ASA- oder AES-Polymere bekannt sind.

7. Polyvinylchlorid.

8. Mischpolymerisate des Vinylchlorids, welche durch die bekannten Verfahren hergestellt werden können (beispielsweise Suspensions-, Masse- oder Emulsionspolymerisation).

9. Mischpolymere des Vinylchlorids mit bis zu 30 Gew.-% an Comonomeren, wie beispielsweise Vinylacetat, Vinylidenchlorid, Vinylether, Acrylnitril, Acrylsäureester, Maleinsäuremono- oder -diester oder Olefinen, sowie Pfropfpolymerisate des Vinylchlorids.

10. Halogenhaltige Polymere, wie beispielsweise Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie beispielsweise, Polyvinylidenchlorid, Polyvinylfluorid, Polyvinylidenfluorid; sowie deren Copolymere wie von Vinylchlorid-Vinylidenchlorid,Vinylchlorid-Vinylacetat oder Vinylidenchlorid-Vinylacetat.

11. Polymere, die sich von $\alpha$-$\beta$-ungesättigten Säuren und deren Derivaten ableiten, wie Polyacrylate und Polymethacrylate, Polyacrylamide und Polyacrylnitrile.

12. Copolymere der unter 11) genannten Monomeren untereinander oder mit anderen ungesättigten Monomeren, wie beispielsweise Acrylnitril-Butadien-Copolymere, Acrylnitril-Alkylacrylat-Copolymere, Acrylnitril-Alkoxyacrylat-Copolymere, Acrylnitril-Vinylhalogenid-Copolymere oder Acrylnitril-Alkylmethacrylat-Butadien-Copolymere.

13. Polymere, die sich von ungesättigten Alkoholen und Aminen bzw. deren Acylderivaten oder Acetalen ableiten, wie Polyvinylalkohol, Polyvinylacetat, - stearat, -benzoat, -maleat, Polyvinylbutyral, Polyallylphthalat, Polyallylmelamin.

14. Homo- und Copolymere von cyclischen Ethern, wie Polyethylenglykole, Polyethylenoxyd, Polypropylenoxyd oder deren Copolymere mit Bisglycidylethern.

15. Polyacetale, wie Polyoxymethylen, sowie solche Polyoxymethylene, die Comonomere wie beispielsweise Ethylenoxyd enthalten.

16. Polyphenylenoxyde und -sulfide und deren Mischungen mit Styrolpolymeren.

17. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte (Polyisocyanate-Polyole-Prepolymere).

18. Polyamide und Copolyamide, die sich von Diaminen und Dicarbonsäuren und/oder von Aminocarbonsäuren oder den entsprechenden Lactamen ableiten, wie Polyamid-4, Polyamid-6, Polyamid-6.6, Polyamid-6.10, Polyamid-11, Polyamid-12, Poly-2,4,4-trimethylhexamethylenterephthalamid, Poly-m-phenylenisophthalamid, sowie deren Copolymere mit Polyethern, wie beispielsweise mit Polyethylenglykol, Polypropylenglykol oder Polytetramethylenglykol.

19. Polyharnstoffe, Polyimide und Polyamid-imide.

20. Polyester, die sich von Dicarbonsäuren und Diolen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-(2,2-bis-(4-hydroxyphenyl)-propan)-terephthalat, Polyhydroxybenzoate, sowie Block-Polyether-ester, die sich von Polyethylen mit Hydroxyendgruppen, Dialkoholen und Dicarbonsäuren ableiten.

21. Polycarbonate und Polyestercarbonate.

22. Polysulfone, Polyethersulfone und Polyetherketone.

23. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

24. Trocknende und nicht trocknende Alkydharze.

25. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

26. Vernetzbare Acrylharze, die sich von substituierten Acrylsäureestern ableiten, wie beispielsweise Epoxyacrylaten, Urethan-acrylaten oder Polyester-acrylaten.

27. Alkydharze, Polyesterharze und Acrylatharze, die mit Melaminharzen, Harnstoffharzen, Polyisocyanaten oder Epoxidharzen vernetzt sind.

28. Vernetzbare Epoxidharze, die sich von Polyepoxiden ableiten, beispielsweise von Bis-glycidylethern oder von cycloaliphatischen Diepoxiden.

29. Natürliche Polymere, wie Cellulose, Naturkautschuk, Gelatine sowie deren polymerhomolog chemisch abgewandelte Derivate, wie Celluloseacetate, -propionate und -butyrate, oder Celluloseether, wie Methylcellulose.

30. Mischungen der oben erwähnten Polymeren, wie beispielsweise PP/EPDM, Polyamid-6/EPDM oder ABS, PVC/EVA, PVC/ABS, PVC/MBS, PC/ABS, PBTP/ABS, PC/ASA, PC/PBT, PVC/CPE, PVD/Acrylat, POM/thermoplastisches PUR, POM/Acrylat, POM/MBS, PPE/HIPS, PPE/Polyamid-6.6 und Copolymere, PA/HDPE, PA/PP, PA/PPE.

31. Natürlich vorkommende und synthetische organische Stoffe, welche reine Monomere oder Mischungen von Monomeren sind, wie beispielsweise Mineralöle, tierische und pflanzliche Fette, Öle und Wachse, oder Öle, Fette und Wachse auf Basis synthetischer Ester oder Mischungen dieser Stoffe.

32. Wäßrige Dispersionen von Natur- oder Synthesekautschuk.

Die zu stabilisierenden organischen Polymeren können noch weitere Zusatzstoffe enthalten, beispielsweise noch folgende Antioxidantien:

1.Alkylierte Monophenole, beispielsweise
2,6-Di-t-butyl-4-methylphenol, 2-t-Butyl-4,6-dimethylphenol, 2,6-Di-t-butyl-4-ethylphenol, 2,6-Di-t-butyl-4-i-butylphenol, 2,6-Di-cyclopentyl-4-methylphenol, 2-($\alpha$-Methylcyclohexyl)-4,6-dimethylphenol, 2,6-Di-octadecyl-4-methylphenol, 2,4,6-Tri-cyclohexylphenol, 2,6-Di-t-butyl-4-methoxymethylphenol.

2.Alkylierte Hydrochinone, beispielsweise
2,6-Di-t-butyl-4-methoxyphenol, 2,5-Di-t-butyl-hydrochinon, 2,5-Di-t-amylhydrochinon, 2,6-Diphenyl-4-octadecyloxyphenol.

3.Hydroxylierte Thiodiphenylether, beispielsweise
2,2'-Thio-bis-(6-t-butyl-4-methylphenol), 2,2'-Thio-bis-(4-octylphenol), 4,4'-Thio-bis-(6-t-butyl-3-methylphenol), 4,4'-Thio-bis-(6-t-butyl-2-methylphenol).

4.Alkyliden-Bisphenole, beispielsweise
2,2'-Methylen-bis-(6-t-butyl-4-methylphenol), 2,2'-Methylen-bis(6-t-butyl-4-ethylphenol), 2,2'-Methylen-bis-[4-methyl-6-($\alpha$-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-t-butylphenol), 2,2'-Ethyliden-bis-(4,6-di-t-butylphenol), 2,2'-Ethyliden-bis-(6-t-butyl-4-isobutylphenol), 2,2'-Methylen-bis-[6-($\alpha$-methylbenzyl)-4-nonylphenol], 2,2'-Methylen-bis-[6-($\alpha$,$\alpha$-dimethylbenzyl)-4-nonylphenol], 4,4'-Methylen-bis-(2,6-di-t-butylphenol), 4,4'-Methylen-bis(6-t-butyl-2-methylphenol), 1,1-Bis-(5-t-butyl-4-hydroxy-2-methylphenyl)-butan, 2,6-Di-(3-t-butyl-5-methyl-2-hydroxybenzyl)-4-methylphenol, 1,1,3-Tris-(5-t-butyl-4-hydroxy-2-methylphenyl)-butan, 1,1-Bis-(5-t-butyl-4-hydroxy-2-methylphenyl)-3-n-dodecylmercaptobutan, Di-(3-t-butyl-4-hydroxy-5-methylphenyl)-dicyclo-pentadien, Di-[2-(3'-t-butyl-2'-hydroxy-5'-methyl-benzyl)-6-t-butyl-4-methyl-phenyl]-terephthalat,
Ethylenglykol-bis-[3,3-bis-(3'-t-butyl-4'-hydroxyphenyl)-butyrat].

5.Benzylverbindungen, beispielsweise
1,3,5-Tri-(3,5-di-t-butyl-4-hydroxybenzyl)-2,4,6-trimethylbenzol, Di-(3,5-di-t-butyl-4-hydroxybenzyl)-sulfid, 3,5-Di-t-butyl-4-hydroxybenzyl-mercaptoessigsäure-isooctylester, Bis-(4-t-butyl-3-hydroxy-2,6-dimethylbenzyl)-dithiol-terephthalat, 1,3,5-Tris-(3,5-di-t-butyl-4-hydroxybenzyl)-isocyanurat, 1,3,5-Tris-(4-t-butyl-3-hydroxy-2,6-dimethylbenzyl)-isocyanurat, 3,5-Di-t-butyl-4-hydroxybenzylphosphonsäure-dioctadecylester, Calciumsalz des 3,5-Di-t-butyl-4-hydroxybenzylphosphonsäure-mono-ethylesters.

6.Acylaminophenole, beispielsweise
4-Hydroxy-laurinsäureanilid, 4-Hydroxy-stearinsäureanilid, 2,4-Bis-octylmercapto-6-(3,5-di-t-butyl-4-hydroxy-anilino)-s-triazin, N-(3,5-Di-t-butyl-4-hydroxyphenyl)-carbaminsäure-octylester.

7.Ester der $\beta$-(3,5-Di-t-butyl-4-hydroxyphenyl)-propionsäure mit ein- oder mehrwertigen Alkoholen, wie z.B. mit
Methanol, Diethylenglykol, Octadecanol, Triethylenglykol, 1,6-Hexandiol, Pentaerythrit, Neopentylglykol, Tris-hydroxyethyl-isocyanurat, Thiodiethylenglykol, Di-hydroxyethyl-oxalsäurediamid.

8.Ester der $\beta$-(5-t-Butyl-4-hydroxy-3-methylphenyl)-propionsäure mit ein- oder mehrwertigen Alkoholen, wie z.B. mit
Methanol, Diethylenglykol, Octadecanol, Triethylenglykol, 1,6-Hexandiol, Pentaerythrit, Neopentylglykol, Tris-hydroxyethyl-isocyanurat, Thiodiethylenglykol, Di-hydroxyethyl-oxalsäurediamid.

9.Amide der $\beta$-(3,5-Di-t-butyl-4-hydroxyphenyl)-propionsäure, wie z.B. N,N'-Di-(3,5-di-t-butyl-4-hydroxyphenylpropionyl)-hexamethylendiamin,N,N'-Di-(3,5-di-t-butyl-4-hydroxyphenylpropionyl)-trimethylendiamin, N,N'-Di-(3,5-di-t-butyl-4-hydroxyphenylpropionyl)-hydrazin.

Daneben können die zu stabilisierenden Polymeren noch weitere Additive enthalten, wie beispielsweise:

1.UV-Absorber und Lichtschutzmittel

1.1 2-(2'-Hydroxyphenyl)-benztriazole, wie z.B. das 5'-Methyl-, 3',5'-Di-t-butyl, 5'-t-Butyl-, 5'-(1,1,3,3-Tetramethylbutyl)-, 5-Chlor-3',5'-di-t-butyl, 5-Chlor-3'-t-butyl-5'-methyl-, 3'-sec.-Butyl-5'-t-butyl-, 4'-Octoxy-, 3',5'-Di-t-amyl-, 3',5'-Bis(α,α-dimethylbenzyl)-Derivat.

1.2 2-Hydroxybenzophenone, beispielsweise das 4-Hydroxy-, 4-Methoxy, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

1.3 Ester von gegebenenfalls substituierten Benzoesäuren, beispielsweise 4-t-Butyl-phenylsalicylat, Phenylsalicylat, Octylphenylsalicyalat, Dibenzoylresorcin, Bis-(4-t-butylbenzoyl)resorcin, Benzoylresorcin, 3,5-Di-t-butyl-4-hydroxybenzoesäure-2,4-di-t-butylphenylester, 3,5-Di-t-butyl-4-hydroxybenzoesäure-hexadecylester.

1.4 Acrylate, beispielsweise
α-Cyan-β,β-diphenylacrylsäure-ethylester bzw. -iso-octylester, α-Carbomethoxyzimtsäuremethylester, α-Cyano-β-methyl-p-methoxyzimtsäuremethylester bzw. -butylester, α-Carbomethoxy-p-methoxyzimtsäure-methylester,N-(β-Carbomethoxy-9-cyano-vinyl)-2-methyl-indolin.

1.5 Nickelverbindungen, beispielsweise
Nickelkomplexe des 2,2'-Thio-bis-[4-(1,1,3,3-tetramethyl-butyl)-phenols], wie der 1:1- oder 1:2-Komplex, gegebenenfalls mit zusätzlichen Liganden wie n-Butyl-amin, Triethanolamin oder N-Cyclohexyldiethanolamin, Nickelalkyl-dithiocarbamate, Nickelsalze von 4-Hydroxy-3,5-di-t-butyl-benzylphosphonsäure-mono-alkylestern wie vom Methyl- oder Ethylester, Nickelkomplexe von Ketoximen wie vom 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden, Nickelsalze der 2-Hydroxy-4-alkoxybenzophenone.

1.6 Sterisch gehinderte Amine, beispielsweise

1.6.1 Bis(2,2,6,6-tetramethylpiperidyl)-sebacat, Bis-(1,2,2,6,6-pentamethylpiperidyl)-sebacat, Bis-(2,2,6,6-tetramethylpiperidyl)-glutarat, Bis-(1,2,2,6,6-pentamethylpiperidyl)-glutarat, Bis-(2,2,6,6-tetramethylpiperidyl)-succinat,
Bis-(1,2,2,6,6-pentamethylpiperidyl)-succinat, 4-Stearyloxy-2,2,6,6-tetramethylpiperidin, 4-Stearyloxy-1,2,2,6,6-pentamethylpiperidin, 4-Stearoyloxy-2,2,6,6-tetramethylpiperidin, 4-Stearoyloxy-1,2,2,6,6-pentamethylpiperidin, 2,2,6,6-Tetramethylpiperidylbehenat, 1,2,2,6,6-Pentamethylpiperidylbehenat, 2,2,4,4-Tetramethyl-7-oxa-3,20-diazadispiro-[5.1.11.2]-heneicosan-21-on, 2,2,3,4,4-Pentamethyl-7-oxa-3,20-diazadispiro-[5.1.11.2]-heneicosan-21-on, 2,2,4,4-Tetramethyl-3-acetyl-7-oxy-3,20-diaza-dispiro-[5.1.11.2]-heneicosan-21-on,
2,2,4,4-Tetramethyl-7-oxa-3,20-diaza-20-(β-lauryloxy-carbonylethyl)-21-oxo-dispiro-[5.1.11.2]-heneicosan, 2,2,3,4,4-Pentamethyl-7-oxa-3,20-diaza-20-(β-lauryloxy-carbonylethyl)-21-oxo-dispiro-[5.1.11.2]-heneicosan, 2,2,4,4-Tetramethyl-3-acetyl-7-oxa-3,20-diazo-20-(β-lauryloxycarbonyl-ethyl)-21-oxo-dispiro-[5.1.11.2]-heneicosan,
1,1',3,3',5,5'-Hexahydro-2,2',4,4',6,6'-hexaaza-2,2',6,6'-bismethano-7,8-dioxo-4,4'-bis-(1,2,2,6,6-pentamethyl-4-piperidyl)biphenyl,N,N',N'',N'''-Tetrakis-[2,4-bis-[N-(2,2,6,6-tetramethyl-4-piperidyl)-butylamino]-1,3,5-triazin-6-yl]-4,7-diazadecan-1,10-diamin, N,N',N'',N'''-Tetrakis[2,4-bis-[N(1,2,2,6,6-pentamethyl-4-piperidyl)-butylamino]-1,3,5-triazin-6-yl]-4,7-diazadecan-1,10-diamin, N,N',N'',N'''-Tetrakis-[2,4-bis-[N-(2,2,6,6-tetramethyl-4-piperidyl)-methoxypropylamino]-1,3,5-triazin-6-yl]-4,7-diazadecan-1,10-diamin, N,N',N'',N'''-Tetrakis-[2,4-bis-[N-(1,2,2,6,6-pentamethyl-4-piperidyl)-methoxypropylamino]-1,3,5-triazin-6-yl]-4,7-diazadecan-1,10-diamin,
Bis-(1,2,2,6,6-pentamethyl-piperidyl)-n-butyl-3,5-di-t-butyl-4-hydroxy-benzylmalonat,
Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Ethandiyl)-bis-(3,3,5,5-tetramethyl-piperazinon).

1.6.2 Poly-N,N'-bis(2,2,6,6-tetramethyl-4-piperidyl)-1,8-diazadecylen, Kondensationsprodukt aus 1-(2-Hydroxyethyl)-2,2,6,6-tetramethyl-4-hydroxy-piperidin und Bernsteinsäure, Kondensationsprodukt aus N,N'-Bis-(2,2,6,6-tetramethyl-4-piperidyl)-hexamethylendiamin und 4-t-Octylamino-2,6-dichlor-1,3,5-triazin, Kondensationsprodukt aus N,N'-Bis-(2,2,6,6-tetramethyl-4-piperidyl)-hexamethylendiamin und 4-Morpholino-2,6-dichlor-1,3,5-triazin.

1.7 Oxalsäurediamide, beispielsweise
4,4'-Di-octyloxy-oxanilid, 2,2'-Di-octyloxy-5,5'-di-t-butyl-oxanilid, 2,2'-Didodecyloxy-5,5'-di-t-butyloxanilid, 2-Ethoxy-2'-ethyl-oxanilid, N,N'-Bis-(3-dimethylaminopropyl)-oxalamid, 2-Ethoxy-5-t-butyl-2'-ethyloxanilid und dessen Gemisch mit 2-Ethoxy-2'-ethyl-5,4-di-t-butyl-oxanilid, Gemische von o- und p-Methoxy- sowie von o- und p-Ethoxy-di-substituierten Oxaniliden.

2. Metalldesaktivatoren, beispielsweise
N,N'-Diphenyloxalsäurediamid, N-Salicylyl-N'-salicyloyl-hydrazin, N,N'-Bis-salicyloylhydrazin, N,N'-Bis-(3,5-di-t-butyl-4-hydroxyphenylpropionyl)-hydrazin, 3-Salicyloylamino-1,2,3-triazol, Bis-benzyliden-oxalsäuredihydrazid.

3. Phosphite und Phosphonite, beispielsweise

Triphenylphosphit, Diphenylalkylphosphite, Phenyldialkylphosphite, Trisnonylphenylphosphit, Trilaurylphosphit, Trioctadecylphosphit, Distearylpentaerythrityldiphosphit, Tris-(2,4-di-t-butylphenyl)-phosphit, Diisodecyl-pentaerythrityldiphosphit, Bis-(2,4-di-t-butylphenyl)-pentaerythrityl-diphosphit, Tristearylsorbityltriphosphit, Tetrakis-(2,4-di-t-butylphenyl)-4,4'-biphenylen-diphosphonit, 3,9-Bis-(2,4-di-t-butylphenoxy)-2,4,8,10-tetraoxa-3,9-diphosphaspiro-[5.5]-undecan, Tris-(2-t-butyl-4-thio-(2'-methenyl-4'-hydroxy-5'-t-butyl)-phenyl-5-methenyl)-phenylphosphit.

4. Peroxidzerstörende Verbindungen, beispielsweise

Ester der $\beta$-Thio-dipropionsäure, wie beispielsweise der Lauryl-, Stearyl-, Myristyl- oder Tridecylester, Mercaptobenzimidazol, das Zinksalz des 2-Mercapto-benzimidazols, Zink-alkyl-dithiocarbamate, Dioctadecylsulfid, Dioctadecyldisulfid, Pentaerythrit-tetrakis-($\beta$-dodecylmercapto)-propionat.

5. Basische Co-Stabilisatoren, beispielsweise

Melamin, Polyvinylpyrrolidon, Dicyandiamid, Triallylcyanurat, Harnstoff-Derivate, Hydrazin-Derivate, Amine, Polyamine, Polyurethane, Alkali- und Erdalkalisalze höherer Fettsäuren oder Phenolate, beispielsweise Ca-Stearat, Zn-Stearat, Mg-Stearat, Na-Ricinoleat, K-Palmitat, Antimonbrenzcatechinat oder Zinnbrenzcatechinat, Hydroxide und Oxide von Erdalkalimetallen oder des Aluminiums, beispielsweise CaO, MgO, ZnO.

6. Nukleierungsmittel, beispielsweise

4-t-Butylbenzoesäure, Adipinsäure, Diphenylessigsäure, Dibenzylidensorbitol.

7. Füllstoffe und Verstärkungsmittel, beispielsweise

Calciumcarbonat, Silikate, Glasfasern, Asbest, Talk, Kaolin, Glimmer, Bariumsulfat, Metalloxide und -hydroxide, Ruß, Graphit.

8. Sonstige Zusätze, beispielsweise

Weichmacher, Gleitmittel, Emulgatoren, Pigmente, optische Aufheller, Flammschutzmittel, Antistatika, Treibmittel.

Die verschiedenen zusätzlichen Additive der vorgenannten Gruppen 1 bis 6 werden den zu stabilisierenden Polymeren in einer Menge von 0,01 bis 10, vorzugsweise 0,01 bis 5 Gew.-%, bezogen auf das Gesamtgewicht der Formmasse, zugesetzt. Der Mengenanteil der Additive der Gruppen 7 und 8 beträgt 1 bis 80, vorzugsweise 10 bis 50 Gew.-%, bezogen auf die gesamte Formmasse.

Die Additive werden nach allgemein üblichen Methoden in die organischen Polymeren eingearbeitet. Die Einarbeitung kann beispielsweise durch Einmischen oder Aufbringen der Verbindungen und gegebenenfalls weiterer Additive in oder auf das Polymere unmittelbar nach der Polymerisation oder in die Schmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglichem Verdunstenlassen des Lösemittels kann die Einarbeitung erfolgen. Die Verbindungen sind auch wirksam, wenn sie in ein bereits granuliertes Polymer nachträglich in einem gesonderten Verarbeitungsschritt eingebracht werden.

Die erfindungsgemäß hergestellten Verbindungen können auch in Form eines Masterbatches, der diese Verbindungen beispielsweise in einer Konzentration von 1 bis 75, vorzugsweise 2,5 bis 30 Gew.-% enthält, den zu stabilisierenden Polymeren zugesetzt werden.

Das erfindungsgemäße Verfahren bietet den Vorteil, daß durch Änderung der Reaktionsführung in einem Mehrphasensystem und Verzicht auf einen Phasentransferkatalysator gleich gute Ergebnisse hinsichtlich Produktqualität und Ausbeute erreicht werden. Außerdem kann die Reaktionszeit von größer 6 Stunden auf 30 Minuten verringert werden. Dies führt zu einer erheblichen Steigerung der Raum-Zeit-Ausbeute und trägt somit zusätzlich zu einer Steigerung der Wirtschaftlichkeit des erfindungsgemäßen Verfahrens bei.

Die nachfolgenden Beispiele und Vergleichsbeispiele dienen der Erläuterung des Erfindungsgegenstandes.

Beispiel 1

2,2,4,4-Tetramethyl-7-oxa-3,20-diaza-20-(2,3-epoxypropyl)-21-oxo-dispiro-[5,1,11,2]-heneicosan und daraus erhaltenes Oligomer.

Zu 180 g Xylol wurden nacheinander 100,0 g (0,25 mol) 2,2,4,4-Tetramethyl-7-oxa-3,20-diaza-21-oxo-dispiro-[5,1,11,2]-heneicosan-Hydrochlorid sowie Epichlorhydrin und Natronlauge in den aus Tabelle 1 zu entnehmenden Mengen gegeben. Diese Mischung wurde 30 Minuten bei 90 bis 95 °C gerührt. Nach dem

Abdestillieren des überschüssigen Epichlorhydrins wurden der Reaktionsmischung 110 g Xylol zugesetzt und die Phasen getrennt. Die organische Phase wurde dreimal mit je 70 g Wasser gewaschen. Nach dem Abdestillieren des Lösemittels im Vakuum erhielt man ein farbloses Öl, welches die in der Überschrift angegebene Epoxyverbindung war. Diese wurde bei 200 °C im Vakuum drei Stunden polymerisiert. Man erhielt ein sprödes, farbloses Harz, dessen Ausbeute und Lösungsviskosität ebenfalls in Tabelle 1 zusammengefaßt sind.

Tabelle 1

| Beispiel | ECh[1] [mol] | NaOH | | Ausbeute [%] | Visk.[2] [$mm^2/s$] |
|---|---|---|---|---|---|
| | | Menge [mol] | Konzentr. [%-ig] | | |
| 1 | 0,50 | 0,75 | 100 | 97 | 1,69 |
| 2 | 0,75 | 0,75 | 100 | 95 | 1,80 |
| 3 | 1,00 | 0,75 | 100 | 96 | 1,87 |
| 4 | 0,50 | 0,91 | 100 | 97 | 1,78 |
| 5 | 0,75 | 0,91 | 100 | 93 | 1,90 |
| 6 | 1,00 | 0,91 | 100 | 96 | 1,82 |
| 7 | 1,25 | 0,91 | 100 | 92 | 1,91 |
| 8 | 1,50 | 0,91 | 100 | 97 | 2,33 |
| 9 | 0,50 | 0,91 | 100 | 94 | 1,46 |
| 10 | 0,75 | 0.91 | 70 | 96 | 1,55 |
| 11 | 1,00 | 0,91 | 70 | 96 | 1,79 |
| 12 | 0,50 | 0,91 | 60 | 95 | 1,61 |
| 13 | 0,75 | 0,91 | 60 | 96 | 1,71 |
| 14 | 1,00 | 0,91 | 60 | 96 | 1,82 |
| 15 | 0,50 | 0,91 | 50 | 95 | 1,44 |
| 16 | 0,75 | 0,91 | 50 | 94 | 1,52 |
| 17 | 1,00 | 0,91 | 50 | 95 | 1,64 |

[1]) Epichlorhydrin
[2]) 20 %ige Lösung in Toluol bei 25 °C nach DGF-M-III 8(75)

Vergleichsbeispiele A bis I

2,2,4,4-Tetramethyl-7-oxa-3,20-diaza-20-(2,3-epoxypropyl)-21-oxo-dispiro-[5,1,11,2]-heneicosan und daraus erhaltenes Oligomer.

Zu 180 g Xylol wurden nacheinander 100,0 g (0,25 mol) 2,2,4,4-Tetramethyl-7-oxa-3,20-diaza-21-oxo-dispiro-[5,1,11,2]-heneicosan-Hydrochlorid, 1,3 g Polyethylenglykol 200 in den Beispielen A bis F bzw. 10 Tropfen Tricaprylammoniumchlorid in den Beispielen G bis I als Phasentransferkatalysator sowie Epichlorhydrin und Natronlauge in aus Tabelle 2 zu entnehmenden Mengen gegeben. Diese Mischung wurde 30 Minuten bei 90 bis 95 °C gerührt. Nach dem Abdestillieren des überschüssigen Epichlorhydrins wurden der Reaktionsmischung 110 g Xylol zugesetzt und die Phasen getrennt. Die organische Phase wurde noch zweimal mit je 70 g Wasser gewaschen. Nach dem Abdestillieren des Lösemittels im Vakuum erhielt man ein farbloses Öl, welches die in der Überschrift angegebene Epoxyverbindung war. Diese wurde bei 200 °C im Vakuum drei Stunden polymerisiert. Man erhielt ein sprödes, farbloses Harz, dessen Ausbeute und Lösungsviskosität in Tabelle 2 wiedergegeben ist.

Tabelle 2

| Vergl.bsp. | ECh [1]) [mol] | NaOH | | Ausbeute [%] | Visk.[2] [$mm^2$/sec] |
|---|---|---|---|---|---|
| | | Menge [mol] | Konz. [%-ig] | | |
| A | 0,50 | 0,75 | 100 | 98 | 1,64 |
| B | 0,75 | 0,75 | 100 | 96 | 1,78 |
| C | 1,00 | 0,75 | 100 | 95 | 1,82 |
| D | 0,50 | 0,91 | 100 | 98 | 1,62 |
| E | 0,75 | 0,91 | 100 | 97 | 1,83 |
| F | 1,00 | 0,91 | 100 | 95 | 1,96 |
| G | 0,50 | 0,91 | 50 | 95 | 1,46 |
| H | 0,75 | 0,91 | 50 | 97 | 1,50 |
| I | 1,00 | 0,91 | 50 | 93 | 1,62 |

[1]) Epichlorhydrin
[2]) 20%ige Lösung in Toluol bei 25 °C nach DGF-M-III 8(75)

Vergleichsbeispiel J

2,2,4,4-Tetramethyl-7-oxa-3,20-diaza-20-(2,3-epoxypropyl)-21-oxo-dispiro-[5,1,11,2]-heneicosan und daraus erhaltenes Oligomer.

Zu 150 $cm^3$ Toluol wurden nacheinander 24,0 g (0,1 mol) 2,2,4,4-Tetramethyl-7-oxa-3,20-diaza-21-oxo-dispiro-[5,1,11,2]-heneicosan-Hydrochlorid, 18,5 g (0,2 mol) Epichlorhydrin, 5 Tropfen Tricaprylmethylammoniumchlorid (®Aliquat 336 der Fa. Fluka) und 40 g 50%ige Natronlauge (≙ 0,5 mol NaOH) gegeben, worauf man das Reaktionsgemisch 12 Stunden bei 60 °C rührte. Nach dem Abstellen des Rührers bildeten sich zwei klare Phasen, welche getrennt wurden. Die organische Phase wurde dreimal mit 50 $cm^3$ Wasser gewaschen, über 50 g Natriumsulfat getrocknet, 30 min mit 1 g Aktivkohle bei Raumtemperatur verrührt und filtriert. Im Vakuum wurden die flüchtigen Anteile abgezogen. Es blieb ein farbloses Öl zurück, welches die in der Überschrift angegebene Epoxiverbindung war.

Diese wurde drei Stunden auf 200 °C erwärmt und polymerisierte dabei zu einem festen, farblosen Harz vom Fp. 130 bis 184 °C und einer Viskosität von 1,50 $mm^2$/s (nach DGF-M-III 8 (75) in 20%iger Lösung in Toluol bei 25 °C).

Die Beispiele zeigen, daß auf den für das Abwasser und somit für die Umwelt nachteilig wirkenden Phasentransferkatalysator, der nach dem Stand der Technik als notwendig erachtet wird, ohne Verlust an Produktqualität und Ausbeute vorteilhaft verzichtet werden kann. Das erfindungsgemäße Verfahren hat somit den Vorteil, daß es die Umwelt weniger belastet und zusätzlich Rohstoffkosten spart.

## Patentansprüche

1. Verfahren zur Herstellung von Polyalkyl-1-oxa-diazaspirodecan-Verbindungen der Formel I

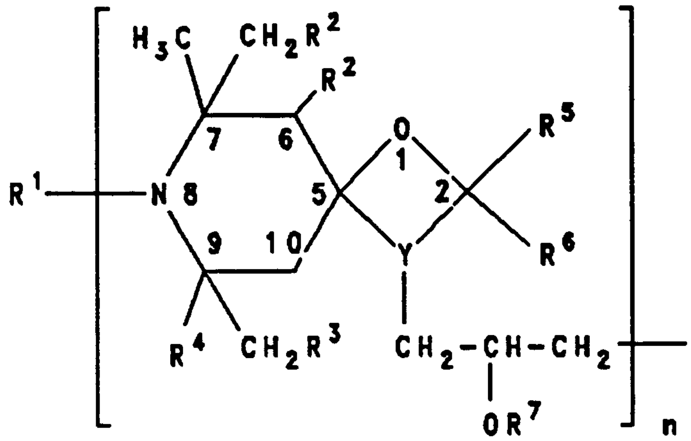

(I),

worin

n eine ganze Zahl von 1 bis 50 ist und

Y die Bedeutung einer Gruppe der Formel II oder III

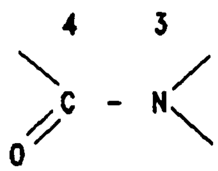

(II)

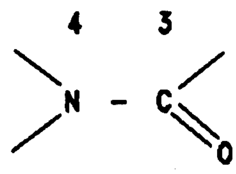

(III)

hat, wobei die Indices 3 bzw. 4 die Ringpositionen im Diazaspirodecansystem angeben und eine Bindung des Stickstoffs mit einer $CH_2$-Gruppe der Propylen-2-oxygruppe verknüpft ist,
$R^1$ ein Wasserstoffatom, ein Sauerstoffatom, eine NO-Gruppe, eine $C_1$-$C_{12}$- - Alkylgruppe, eine Allylgruppe, eine $C_1$-$C_{22}$-Acylgruppe, eine Benzylgruppe, eine $C_1$-$C_{12}$-Alkyloxy-Gruppe oder eine $C_3$-$C_{12}$-Cycloalkoxy-Gruppe,
$R^2$ und $R^3$ entweder gleich sind und ein Wasserstoffatom oder eine $C_1$-$C_5$-Alkylgruppe bedeuten, wobei dann $R^4$ eine Methylgruppe ist, oder
$R^2$ ein Wasserstoffatom oder eine $C_1$-$C_5$-Alkylgruppe ist und $R^3$ und $R^4$ zusammen mit den sie verbindenden Kohlenstoffatomen eine $C_5$- oder $C_6$-Cycloalkylgruppe oder eine Gruppe der Formel

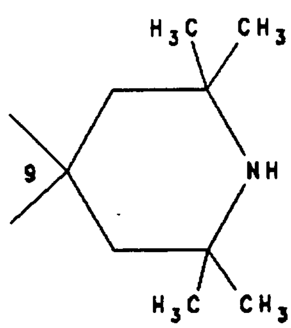

bilden,

$R^5$ und $R^6$ gleich oder verschieden sind und für ein Wasserstoffatom, eine $C_1$-$C_{30}$-, - Alkylgruppe, für eine unsubstituierte oder durch Chlor oder $C_1$-$C_4$-Alkyl substituierte $C_7$-$C_{12}$-Phenylalkylgruppe stehen, oder

$R^5$ und $R^6$ zusammen mit dem sie verbindenden Kohlenstoffatom eine unsubstituierte oder durch bis zu vier $C_1$-$C_4$-Alkylgruppen substituierte $C_5$-$C_{18}$-Cycloalkylgruppe oder eine Gruppe der Formel

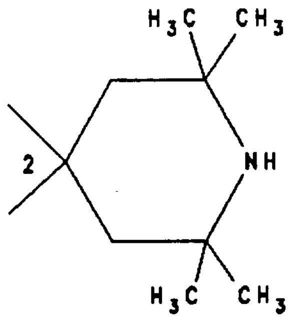

bilden,

$R^7$ bei n = 1 keine Bedeutung hat, sodaß das Sauerstoffatom mit der endständigen $CH_2$-Gruppe verbunden ist und einen Oxiranring bildet,

$R^7$ bei n > 1 ein Wasserstoffatom oder eine $C_1$-$C_{22}$-Acylgruppe ist, oder in der endständigen Monomereneinheit keine Bedeutung hat, so daß das Sauerstoffatom mit der enständigen $CH_2$-Gruppe verbunden ist und einen Oxiranring bildet, durch Umsetzung einer Verbindung der Formel IV

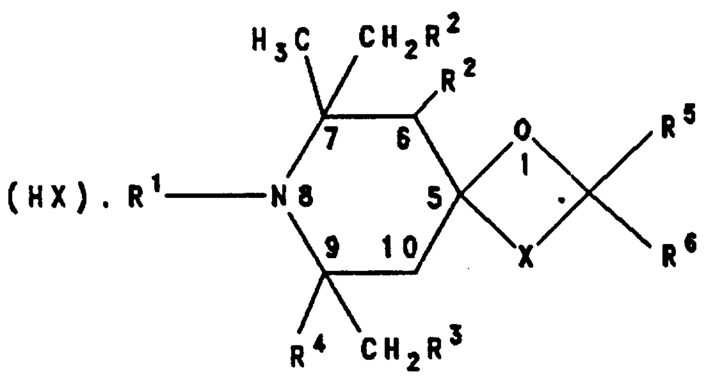

(IV)

worin $R^1$, $R^2$, $R^3$, $R^4$, $R^5$ und $R^6$ die obengenannte Bedeutung haben und (HX) ein Säurerest ist, oder deren Salz mit einer Protonensäure

mit einem Epihalohydrin der Formel V

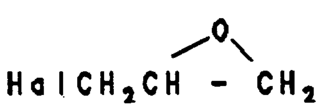

(V)

worin Hal ein Chlor-, Brom- oder Jodatom ist, im Molverhältnis 1:1 bis 1:10 in Anwesenheit eines Alkalihydroxyds in einem inerten organischen Lösemittel und, bei n > 1, Erhitzen der entstandenen Epoxiverbindung VI

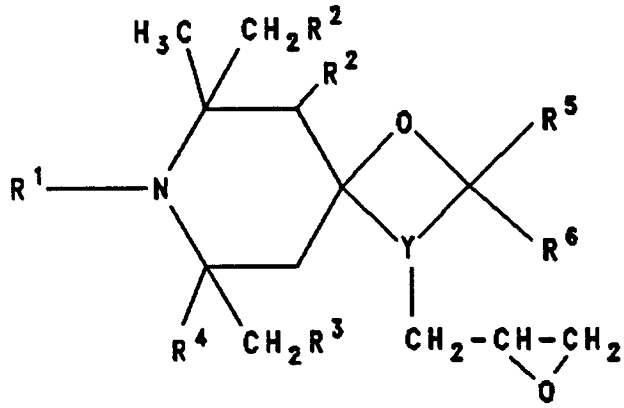

(VI)

worin $R^1$, $R^2$, $R^3$, $R^4$, $R^5$ und $R^6$ die obengenannte Bedeutung haben, auf eine Temperatur von 100 bis 240 °C, dadurch gekennzeichnet, daß die Umsetzung der Verbindung der Formel IV mit der Verbindung der Formel V in Gegenwart der äquimolaren bis zwanzigfach molaren Menge festen Alkalimetallhydroxids oder der entsprechenden Menge einer Mischung aus festem Alkalimetallhydroxid und Wasser im Gewichtsverhältnis 1:9 bis 9:1 als alleinigem Katalysator durchgeführt wird und nach Beendigung der Reaktion dem Gemisch soviel Wasser zugesetzt wird, daß sich zwei Phasen bilden.

**2.** Verfahren nach Anspruch 1, dadurch gekennzeichnet, daß das verwendete inerte Lösemittel Toluol oder Xylol ist.

**3.** Verfahren gemäß Anspruch 1, dadurch gekennzeichnet, daß als Katalysator Natriumhydroxid in fester Form oder im Gemisch mit Wasser verwendet wird.

**4.** Verfahren gemäß Anspruch 1, dadurch gekennzeichnet, daß die Verbindung der Formel IV 2,2,4,4-Tetramethyl-7-oxa-3,20-diaza-21-oxo-dispiro-[5,1,11,2]-heneicosan oder dessen Hydrochlorid ist.

**5.** Verfahren gemäß Anspruch 1, dadurch gekennzeichnet, daß die Verbindung der Formel V Epichlorhydrin ist.

Europäisches Patentamt

# EUROPÄISCHER RECHERCHENBERICHT

Nummer der Anmeldung
EP 95 10 1686

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X,Y | EP-A-0 224 181 (HOECHST AG)<br>* Seite 4, letzte Zeile bis Seite 5, Zeile 18; Seite 5, Zeilen 30-40 *<br>* Seite 6, Zeilen 6-15 und Verbindung Nr. 30 *<br>--- | 1-5 | C07D498/10<br>C07D519/00<br>C08G73/06<br>//(C07D498/10, 263:00, 221:00), (C07D519/00, 498:00,498:00) |
| D,Y | EP-A-0 402 889 (HOECHST AG)<br>* Seite 4, Verbindung (11); Seite 5, Zeilen 1-38; Beispiele 1, 11 *<br>----- | 1-5 | |

RECHERCHIERTE SACHGEBIETE (Int.Cl.6)
C07D
C08K
C08G

Der vorliegende Recherchenbericht wurde für alle Patentansprüche erstellt

| Recherchenort | Abschlußdatum der Recherche | Prüfer |
|---|---|---|
| BERLIN | 8.Mai 1995 | Hass, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C03)